Europäisches Patentamt

European Patent Office

Office européen des brevets

(19)

(11) Veröffentlichungsnummer : **0 119 578**
**B1**

(12)

# EUROPÄISCHE PATENTSCHRIFT

(45) Veröffentlichungstag der Patentschrift :
09.09.87

(21) Anmeldenummer : 84102689.1

(22) Anmeldetag : 12.03.84

(51) Int. Cl.⁴ : **C 12 M   1/06**

(54)   **Vorrichtung zum Züchten von Mikroorganismen in einer Nährsubstratlösung.**

(30) Priorität : 16.03.83 PCT/EP83/00079

(43) Veröffentlichungstag der Anmeldung :
26.09.84 Patentblatt 84/39

(45) Bekanntmachung des Hinweises auf · die Patenterteilung : 09.09.87 Patentblatt 87/37

(84) Benannte Vertragsstaaten :
AT CH DE FR GB IT LI NL SE

(56) Entgegenhaltungen :
GB-A-   423 331
GB-A- 2 032 956
SU-A-   181 039
US-A- 2 094 004
US-A- 4 025 394
Die Akte enthält technische Angaben, die nach dem Eingang der Anmeldung eingereicht wurden und die nicht in dieser Patentschrift enthalten sind.

(73) Patentinhaber : **Zentrale Finanz- und Kommerz-Aktiengesellschaft**
**Postfach 1 42**
**FL-9490 Vaduz (LI)**

(72) Erfinder : **Buchwalder, Gerard**
**50 Rue du Domont**
**CH-2800 Delémont (CH)**

(74) Vertreter : **Patentanwälte Kirschner & Grosse**
**Herzog-Wilhelm-Strasse 17**
**D-8000 München 2 (DE)**

EP 0 119 578 B1

Jouve, 18, rue St-Denis, 75001 Paris, France

## Beschreibung

Die Erfindung betrifft eine Vorrichtung zum Züchten von Mikroorganismen nach dem Oberbegriff des Patentanspruchs 1.

Bei der Züchtung von Mikroorganismen werden die Mikroorganismen in der Regel submers in einer fluiden Nährsubstratlösung suspendiert gehalten, wobei zugleich die für das Wachstum erforderlichen Parameter wie Temperatur, pH-Wert, Sauerstoffkonzentration usw. eingestellt werden. Eine optimale Versorgung der in der Nährsubstratlösung suspendierten Mikroorganismen mit den Wachstums- bzw. Produktionsnotwendigen Substanzen (Kohlenstoffquelle, Salze, Vitamine, Spurenelemente und Sauerstoff) sowie die Entfernung der Abbauprodukte bedingen Züchtungsverhältnisse, die hauptsächlich eine intensive Durchmischung der Nährsubstratlösung und damit gleichmäßige homogene Verteilung der Phasen, eine hohe Gas-Flüssig-Stoffaustauschleistung und gute Zerteilung der Gasphase sowie eine gute Wärmeableitung gestatten. Unter einer homogenen Verteilung ist nicht nur die üblicherweise verstandene Homogenisierung der meist wässrigen Trägerphase bezüglich darin gelöster Substratkomponenten zu verstehen, sondern auch eine vollständige Suspendierung der einzelnen Mikroorganismen in der Nährlösung.

Vorrichtungen zu diesem Zweck sind auch unter dem Begriff Fermenter beispielsweise aus Ullmanns Encyklopädie der technischen Chemie, 4. Auflage, Band 8, Seite 517-519 bekannt. Bei solchen Fermentern handelt es sich im wesentlichen um senkrecht stehende, trommelförmige Behälter, in denen die die Mikrooganismen enthaltende Nährsubstratlösung mittels verschiedenartiger Rührer bewegt wird, wodurch die Mikroorganismen suspendiert und Temperatursowie Konzentrationsverteilung gleichmäßig gehalten werden soll. Es ist aus dieser Druckschrift weiterhin bekannt, zur Erhöhung der Reaktionsfähigkeit der Mikroorganismen sowie zur Bewegung der Nährsubstratlösung dem Fermenter Gase zuzuführen.

Bei all diesen bekannten Fermentern findet die Strömung der Nährsubstratlösung in senkrechter Richtung statt, wobei bei einer Begasung das Gas mit der vom Rührer erzeugten Strömung mitgerissen wird und somit eine Begasung bzw. Belüftung der Nährsubstratlösung entsprechend der bevorzugten Strömungsrichtung ungleichmäßig erfolgt. Bei ungleichmäßiger Strömung im Fermenter treten jedoch dadurch Probleme auf, daß Mikroorganismen dazu neigen, sich in Form dichtgepackter Schichten an der Reaktorwandung in Bereichen geringerer Strömungsintensität abzulagern. Dies führt einerseits zu einer Verarmung der Nährsubstratlösung an aktiven Organismen, andererseits zu einer Produktivitäts- oder Produktqualitätseinbuße, da die die Wandschicht bildenden Mikroorganismen einer Substratlimitierung unterliegen.

Aufgabe der vorliegenden Erfindung ist es daher, eine Vorrichtung zum Züchten von Mikroorganismen in einer Nährsubstratlösung zu schaffen, bei der die im Zuchtbehälter herrschende Strömung gleichmäßig verteilt und eine verbesserte Sauerstoffzufuhr gewährleistet ist.

Erfindungsgemäß wird diese Aufgabe durch eine Vorrichtung mit den Merkmalen gemäß des kennzeichnenden Teils des Anspruchs 1 gelöst.

Weitere vorteilhafte Ausgestaltungen der Erfindung ergeben sich aus den Unteransprüchen.

Durch die erfindungsgemäße Vorrichtung wird im Fermenter eine Kreislaufströmung erzielt, die quer zur Auftriebsrichtung der Gasbläschen verläuft. Die erfindungsgemäße Kreislaufströmung bewirkt eine im Behälter gleichmäßig verteilte Strömung, um die Gefahr der Wandablagerungen zu vermindern. Darüber hinaus bewirkt das als Leitvorrichtung für die Kreislaufströmung wirkende Lochblech eine zusätzliche Zerteilung der Gasbläschen beim Durchtritt durch die Öffnungen, so daß die Gasbläschenzahl erhöht und somit die Durchlüftung verbessert wird. Diese Begasung wird erfindungsgemäß dadurch erhöht, daß die Aufstiegsgeschwindigkeit der Gasbläschen quer zur Strömungsrichtung erfolgt.

Ein Ausführungsbeispiel der Erfindung wird nun anhand der beiliegenden Zeichnungen beschrieben. Es zeigen:

Figur 1 eine schematische Seitenansicht einer erfindungsgemäßen Vorrichtung;

Figur 2 eine schematische Draufsicht auf die erfindungsgemäße Vorrichtung nach Fig. 1; und

Figur 3 eine schematische Frontansicht der erfindungsgemäßen Vorrichtung.

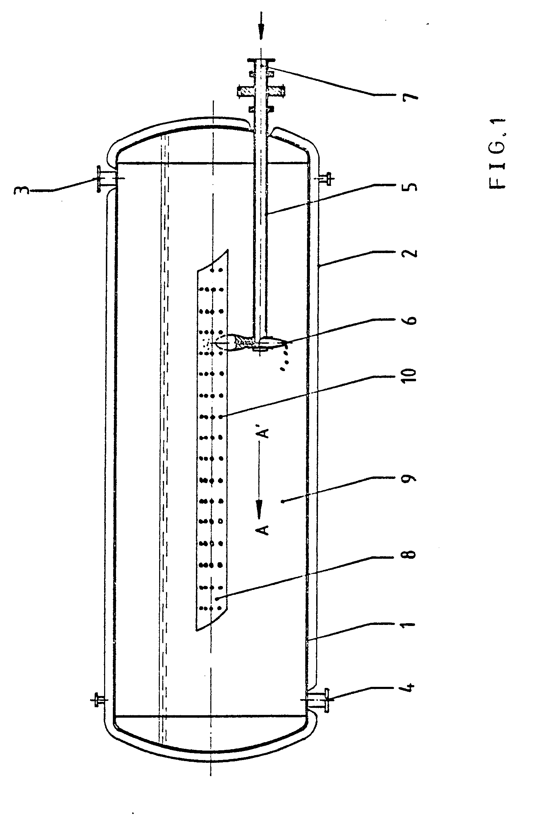

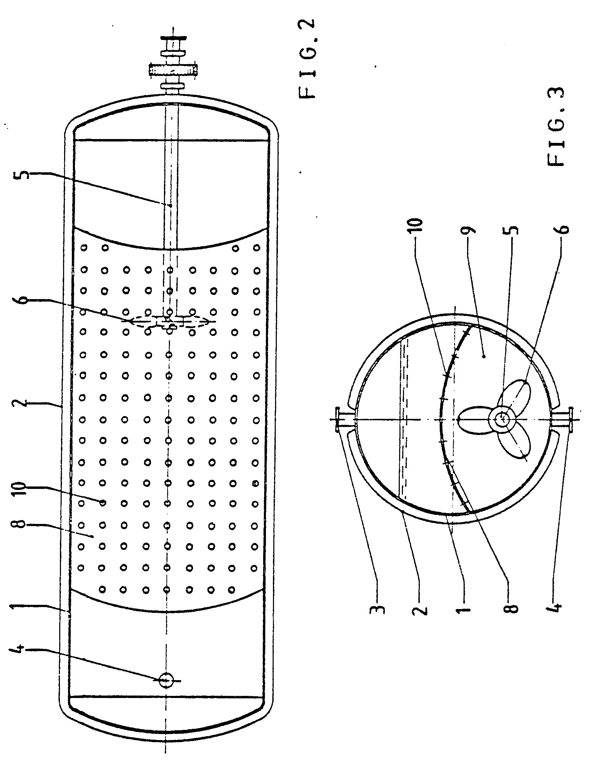

Die in Fig. 1 gezeigte Vorrichtung weist einen horizontal liegenden Behälter 1, z. B. aus Edelstahl, auf, der allseitig von einem Wärmetauschermantel 2 umgeben ist. Der Behälter 1 hat eine zylindrische Seitenwand und abgerundete Böden. Die Abrundung der Böden kann kreisbogenförmig oder elliptisch abgeflacht sein. Der Behälter 1 wird durch einen Stutzen 3 mit Nährsubstratlösung befüllt und mit Mikroorganismen beimpft. Ein Stutzen 4 dient zum Ablassen der Fermentationsbrühe nach beendigter Fermentation. Mittels einer Hohlwelle 5 wird durch einen als Hohlrührer ausgebildeten Rührpropeller 6 sauerstoffhaltiges Gas an einem Gasstutzen 7 eingeleitet und unterhalb einer Leitvorrichtung, die als Lochblech 8 ausgeführt ist, in Strömungsrichtung A, A' in einer Nährsubstratlösung 9 verteilt. Der sich nach oben, d. h. mit einer Bewegungskomponente senkrecht zur Strömungsrichtung der Nährsubstratlösung, bewegende Gasblasenstrom durchströmt, zum Teil das Lochblech 8, wodurch die Gasblasen erneut zerteilt werden, um nochmals die Nährsubstratlösung zu durchströmen, bevor das Restgas durch den Stutzen 3 abgesogen wird. Aus Fig. 2 ist die Anordnung des Lochbleches 8 mit den Löchern 10 ersichtlich. Ferner ist aus dieser Fig. 2 die freie Querschnittsfläche innerhalb des Behälters zu erkennen, in dem die Nährsubstratlösung 9 im Kreislauf strömt. Die freie Querschnittsfläche innerhalb des Behälters 1 entsteht dadurch, daß das Lochblech 8 an der Seitenwandung des Behälters befestigt ist und unter Abstand von den Böden zur Bildung von Durchtrittsöffnungen bzw. freier Querschnittsfläche für die Strömung der Nährsubstratlösung endet. Durch die Durchtrittsöffnungen tritt die Kreislaufströmung von unterhalb des Lochbleches 8 nach oben (linkes Ende des Behälters) bzw. von oberhalb des Lochblechs 8 in den unteren Bereich des Behälters (rechtes Ende des Behälters in Fig. 1). Das Lochblech ist in einer solchen Höhe an der Seitenwandung des Behälters 1 befestigt, daß der Durchflußquerschnitt für die Kreislaufströmung der Nährsubstratlösung oberhalb und unterhalb des Lochbleches 8 gleich groß ist. Aus der Frontansicht von Fig. 3 ist ersichtlich, wie das Lochblech 8 an den Seitenwänden des Behälters 1 befestigt und nach oben gewölbt ist.

Der Durchmesser der Löcher des Lochbleches 8 liegt zwischen 0,5mm und 5mm, vorzugsweise bei 2mm, während der freie Lochquerschnitt des Lochblechs 8 zwischen 15 % und 40 %, vorzugsweise bei 23 %, liegt.

Die Vorteile der erfindungsgemäßen Vorrichtung bei der Züchtung von Mikroorganismen werden im folgenden an Hand eines Vergleiches einer Züchtung in einem herkömmlichen Fermenter mit einer Züchtung in einer erfindungsgemäßen Vorrichtung näher beschrieben.

Züchtung in herkömmlichem Fermenter

Eine Impfkultur von Streptomyces Aureofaciens ergibt im Labor unter optimalen Bedingungen in einem 5-Liter-Fermenter mit Rührturbine und Belüftungsrate von 1,5 Volumeneinheiten Luft pro Volumeneinheit Flüssigkeit pro Minute eine maximale Ausbeute von 18 Gramm Chlortetracyclin pro Liter Nährsubstratlösung.

Bei Übertragung der Züchtung des vorgenannten Mikroorganismus auf einen 3000-Liter-Fermenter, welcher als begaster Rührturbinenreaktor nach der DECHEMA-Betreibernorm (DECHEMA = Deutsche Gesellschaft für Chemisches Apparatewesen eV) ausgeführt ist, wird unter optimierten Bedingungen bei einer Belüftungsrate von 1 Volumeneinheit Luft pro Volumeneinheit Flüssigkeit pro Minute und einer spezifischen Rührleistung von 1,5 Watt/Liter nur eine Maximalausbeute von 12 Gramm Chlortetracyclin/Liter der Nährsubstratlösung erzielt. Der 3000-Liter-Fermenter nach DECHEMA-Betreibernorm ist ein zylindrischer, vertikal stehender Tank mit elliptischen Böden und einem Längen- zu Durchmesserverhältnis von 3,3.

Nach Beendigung der Fermentation wurde der Apparat untersucht und starker Bewuchs im Bereich der Stromstörer festgestellt.

Züchtung in der erfindungsgemäßen Vorrichtung

Eine erfindungsgemäße Vorrichtung mit einem Volumen von 3000 Liter Behälterinhalt, bestehend aus einem liegenden zylindrischen Tank mit elliptischem Boden, ausgeführt nach Fig. 1 mit einem Längen- zu Durchmesserverhältnis von 3,3 wird bei einer spezifischen Rührleistung von 1 Watt/Liter Nährsubstratlösung mit 1 Volumeneinheit Luft pro Volumeneinheit Flüssigkeit pro Minute begast und mit der gleichen Kultur von Streptomyces Aureofaciens wie im Beispiel 1 beimpft. Nach der gleichen Zeit wie im Laborversuch des Beispiels 1 wurde die Fermentation beendet und die Fermentationsbrühe untersucht. Die Ausbeute an Chlortetracyclin betrug 18 Gramm/Liter Nährsubstratlösung. Der Behälter wies keinen Wandbewuchs auf.

Als horziontale Leitvorrichtung diente für die Vorrichtung des Beispiels 2 ein Lochblech mit einer freien Querschnittsfläche von 23 % und einem Lochdurchmesser von 2mm.

**Patentansprüche**

1. Vorrichtung zum Züchten von Mikroorganismen in einer Nährsubstratlösung, die in einem Behälter strömt und begast wird, gekennzeichnet durch eine längliche Ausgestaltung des Behälters (1) zur Aufnahme der Nährsubstratlösung mit den darin suspendierten Mikroorganismen, welcher horizontal gelagert ist und eine Seitenwandung sowie Böden an seiner Vorder- und Rückseite aufweist, eine in die Nährsubstratlösung eintauchende, als Lochblech (8) ausgebildete Leitvorrichtung, die an der Seitenwandung befestigt ist und unter Abstand von den Böden zur Bildung von Durchtrittsöffnungen endet, um eine Kreislaufströmung der Nährsubstratlösung oberhalb und unterhalb des Lochblechs (8) zu gestalten, eine als Propellerrührer (6) ausgebildete Rühreinrichtung für die Nährsubstratlösung in dem Behälter (1), wobei der Propellerrührer (6) unterhalb des Lochblechs (8) zur Erzeugung der Kreislaufströmung angeordnet ist, und eine Einrichtung für die Zufuhr von sauerstoffhaltigem Gas durch eine Hohlwelle (5) des Propellerrührers (6) in die Nährsubstratlösung, wobei das sauerstoffhaltige Gas der Nährsubstratlösung derart verteilt wird, daß es eine Bewegungskomponente senkrecht zur Strömungsrichtung der Nährsubstratlösung in der Kreislaufströmung aufweist.

2. Vorrichtung nach Anspruch 1, dadurch gekennzeichnet, daß der Durchflußquerschnitt für die Strömung der Nährsubstratlösung oberhalb und unterhalb des Lochblechs (8) gleich groß ist.

3. Vorrichtung nach Anspruch 1, dadurch gekennzeichnet, daß die Böden des Behälters (1) abgerundet sind.

4. Vorrichtung nach Anspruch 1, dadurch gekennzeichnet, daß das Lochblech (8) in Frontansicht nach oben gewölbt ist.

5. Vorrichtung nach Anspruch 1 oder 4, dadurch gekennzeichnet, daß der Durchmesser der Löcher des Lochblechs (8) zwischen 0,5 mm und 5 mm, vorzugsweise bei 2 mm liegt.

6. Vorrichtung nach Anspruch 1, dadurch gekennzeichnet, daß der freie Lochquerschnitt des Lochblechs (8) zwischen 15 % und 40 %, vorzugsweise bei 23 % liegt.

### Claims

1. Apparatus for the culture of microorganisms in a liquid nutritious substrate streaming inside a tank and being attacked with gas, characterised by an elongate design of the tank (1) for containing the liquid nutritious substrate with the microorganisms suspended therein, said tank being horizontally positioned and having a tubular wall as well as bottom plates on its front and back sides, a guiding device in form of a perforated sheet metal (8) submerged in the liquid nutritious substrate, which is fastened on the tubular wall and spaced from the bottom plates so as to form pass-through openings in order to provide for circulation of the liquid nutritious substrate above and below the perforated sheet metal (8), a stirring device in form of a propeller stirrer (6) for stirring the liquid nutritious substrate inside the tank (1), said propeller stirrer (6) being arranged below the perforated sheet metal (8) to generate the circulation, and means for the supply of oxygen-containing gas through a hollow shaft (5) of the propeller stirrer (6) into the liquid nutritious substrate, said oxygen-containing gas in the liquid nutritious substrate being diffused in a manner that it has a motion component perpendicular to the flow direction of the liquid nutritious substrate in the circulation.

2. Apparatus according to claim 1, characterised in that the cross-section of passage for the flow of the liquid nutritious substrate above the perforated sheet metal (8) is the same as below the perforated sheet metal.

3. Apparatus according to claim 1, characterised in that the bottom plates of the tank (1) are rounded.

4. Apparatus according to claim 1, characterised in that in the front view the perforated sheet metal (8) is upwardly vaulted.

5. Apparatus according to claim 1 or 4, characterised in that the diameter of the holes of the perforated sheet metal (8) is between 0.5 and 5 mm, preferably 2 mm.

6. Apparatus according to claim 1, characterised in that the free cross-section of the holes of the perforated sheet metal (8) is between 15 and 40 %, preferably 23 %.

### Revendications

1. Installation pour la culture de micro-organismes dans une solution de substrat nutritif coulant dans un récipient et alimenté par un gaz, caractérisée par une façon oblongue du récipient (1) pour recevoir la solution de substrat nutritif avec les micro-organismes suspendus dedans, le récipient étant logé horizontalement et présentant une paroi latérale ainsi que des fonds à ses faces avant et arrière, un dispositif de guidage sous la forme d'une tôle perforée (8), plongé dans la solution de subtrat nutritif, fixé à la paroi latérale et se terminant à une distance des fonds de manière à laisser des ouvertures de passage dans le but de faire circuler la solution de substrat nutritif au-dessus et au-dessous de la tôle perforée (8), un agitateur sous la forme d'un agitateur à hélice (6) pour la solution de substrat nutritif dans le récipient (1), l'agitateur à hélice (6) étant arrangé au-dessous de la tôle perforée (8) afin de générer l'écoulement circulaire, et une installation pour l'alimentation d'un gaz oxygénifère à travers un arbre creux (5) de l'agitateur à hélice (6) dans la solution de substrat nutritif, le gaz oxygénifère de la solution de substrat nutritif étant répandu de manière qu'il présente une composante de mouvement perpendiculaire au sens d'écoulement de la solution de substrat nutritif dans l'écoulement circulaire.

2. Installation selon la revendication 1, caractérisée en ce que les sections d'écoulement de la solution de substrat nutritif au-dessus et au-dessous de la tôle perforée (8) sont identiques.

3. Installation selon la revendication 1, caractérisée en ce que les fonds du récipient (1) sont arrondis.

4. Installation selon la revendication 1, caractérisée en ce que la tôle (8), en vue de face, est voûtée vers le haut.

5. Installation selon la revendication 1 ou 4, caractérisée en ce que le diamètre des perforations de la tôle perforée (8) est de 0,5 mm à 5 mm, de préférence de 2 mm.

6. Installation selon la revendication 1, caractérisée en ce que la coupe en travers libre des perforations de la tôle perforée (8) est de 15 % à 40 %, de préférence de 23 %.

FIG.1

0 119 578

FIG. 2

FIG. 3